# EUROPEAN PATENT SPECIFICATION

(11) **EP 1 365 807 B1**
(45) Date of publication and mention of the grant of the patent: **08.06.2005**
(21) Application number: 01983892.9
(22) Date of filing: 16.11.2001
(51) Int. Cl.: A61K 45/00, A61F 5/44, A61F 2/04

(54) **RECEPTACLE**
BEHÄLTNIS
RECEPTACLE

(30) Priority: 20.11.2000 SE 0004250
(43) Date of publication of application: 03.12.2003
(73) Proprietor: SINOVA - Safetech Innovation Aktiebolag, 216 22 Limhamn (SE)
(72) Inventor: UNO, Jörgen, S-216 22 Limhamn (SE)
(74) Representative: Wallengren, Yngvar
(86) International application number: PCT/SE2001/002547
(87) International publication number: WO 2002/039959

(56) References cited:
- US-A- 5 108 430
- US-A- 5 246 456
- US-A- 5 279 600
- US-A- 5 352 183
- US-A- 6 050 982

## Description

### TECHNICAL FIELD

The present invention relates to a receptacle for the collection of excrement and designed for implant in the abdominal cavity of a patient who, post-operatively, lacks the whole or part of the small intestine, large intestine or the rectum, the receptacle comprising a connection device for connection to the intestine of the patient and an emptying device which realises a closable passage through the abdominal wall of the patient and is fixed therein.

### BACKGROUND ART

Many intestinal diseases entail an operative removal of all or parts of the small intestine and/or the large intestine and also the rectum, often as a result of cancer. In order thereafter to be able to make possible emptying of the intestine, a colostomy is applied today. This entails that the remaining end of the intestine after this operation is moved by operation to an external skin surface, whereafter the contents of the intestine are collected in a colostomy pouch. This involves major problems for many patients, such as the constant worry about leakage and odour which in many cases causes discomfort in stressful public meetings, presentations etc., in working life. Social life will also be inhibited, such as at dinners, baths, saunas, not to mention sexual performance. The private feeling of never being clean, of constantly carrying a small bag packed with colostomy pouches etc., as well as changes of clothing, are major difficulties. In addition, there are medical drawbacks, such as hernias, problems involving omentum majus et minor, skin irritations, eczema, necrosis etc.

### PROBLEM STRUCTURE

The present invention has for its object to design the receptacle intimated by way of introduction such that the drawbacks inherent in prior art technology and methods are obviated.

The object forming the basis of the present invention will be attained if the receptacle intimate by way of introduction is given the characterising features as set forth in appended Claim 1.

### BRIEF DESCRIPTION OF THE ACCOMPANYING DRAWINGS

The present invention will now be described in greater detail hereinbelow with reference to the accompanying Drawings. In the accompanying Drawings:
- Fig. 1: is a view straight from the side of the subject matter of the present invention implanted in a patient:
- Fig. 2: is a view corresponding to that of Fig. 1, but seen straight from the front;
- Fig. 3: shows the receptacle according to the present invention seen separately;
- Fig. 4: shows those devices by means of which the subject matter of the present invention is secured in the patient's abdominal cavity;
- Fig. 5: shows an emptying device included in the subject matter of the present invention;
- Fig. 6: is a further view of the emptying device according to Fig. 5;
- Fig. 7: shows the emptying device according to Figs. 5 and 6 and an evacuation tube with a filter included therein;
- Fig. 8: shows the emptying device in the permanently operated state in a first embodiment;
- Fig. 9: is a view corresponding to that of Fig. 8 of an alternative embodiment;
- Fig. 10: shows a connection device included in the subject matter of the present invention;
- Fig. 11: shows a communication tube to the interior of the abdominal cavity included in the emptying device according to Fig. 5;
- Fig. 12: shows a catheter designed for emptying the receptacle;
- Fig. 13: is a view straight from the front showing emptying of the subject matter of the present invention; and
- Fig. 14: shows the catheter according to Figs. 12 and 13 with its package.

### DESCRIPTION OF PREFERRED EMBODIMENT

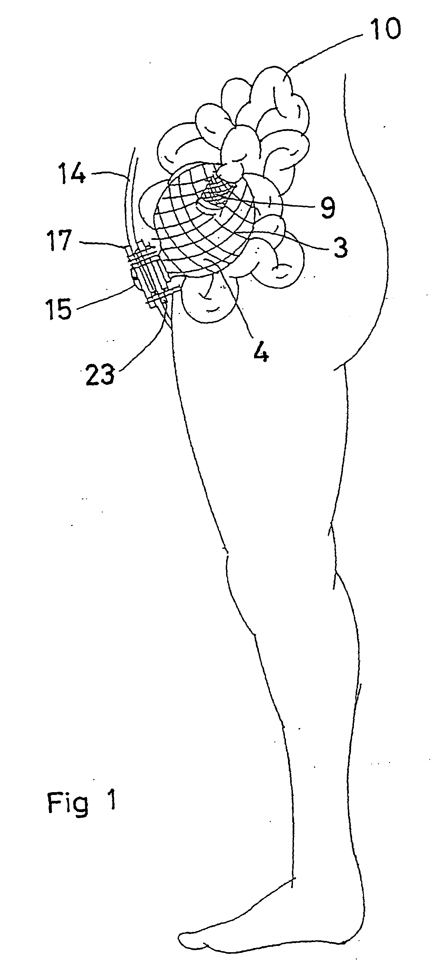

It will be apparent from Fig. 1 that the receptacle according to the present invention is operated into the lower part of the patient's abdominal cavity inside the abdominal wall 14. The receptacle consists of two main components, an inner container or ampoule 4 of a gas- and liquid-tight material which is flexible or possibly somewhat elastic, and an outer envelope or casing 3 which is produced from a flexible but substantially unstretchable material. As an example of suitable materials for the ampoule 4, mention might be made of a silicon material, and as regards the outer casing, titanium or a titanium alloy. The outer casing 3 is in the form of a net which encloses the ampoule 4. At the rear/upper end of the receptacle, there is disposed a connection device which int. al. includes an interjacent member 9 which places the interior of the receptacle in communication with the intestine 10.

In the front/lower end of the receptacle, there is provided an emptying device which int. al. includes an outer fixing plate 17, an inner fixing plate 23 and a lid 15. A more detailed description of the emptying device and the connection device will be given below.

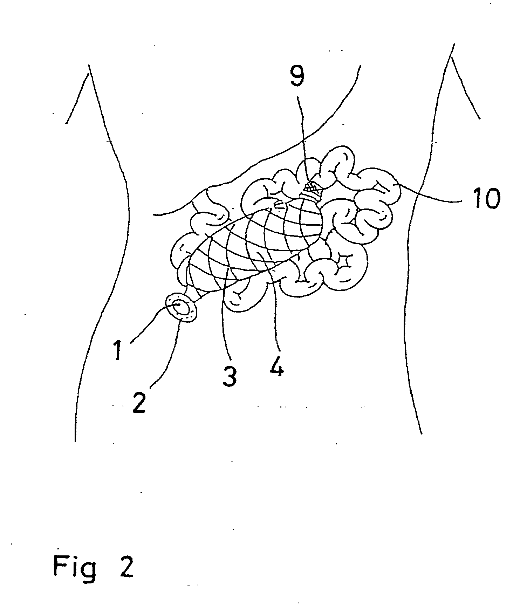

As is apparent from Figs. 1 and 2 together, the receptacle is elongate with approximately twice as great a length as diameter or transverse dimension. The configuration is approximately an ellipsoid, but may also deviate from this basic configuration. The receptacle has a longitudinal axis which, in the sagittal plane, inclines approximately 35-45° to the horizontal. As will be described in greater detail below, the receptacle is fixed, e.g. in omentum majus et minor or alternatively in the muscular structure of the rear abdominal wall. However, this fixing is not absolute, but must permit a certain possibility of movement of the order of magnitude of 3-4° in all- directions. Further, the front/lower end of the ampoule is fixed in the abdominal wall via the fixing plates 17 and 23.

In Fig. 2, reference numeral 1 relates to the front opening of the receptacle via which the contents of the intestine may pass from the receptacle to the ambient surroundings outside the abdominal wall 14. Reference numeral 2 relates to a front connection to the receptacle, by means of which connection the receptacle is connected to the emptying device.

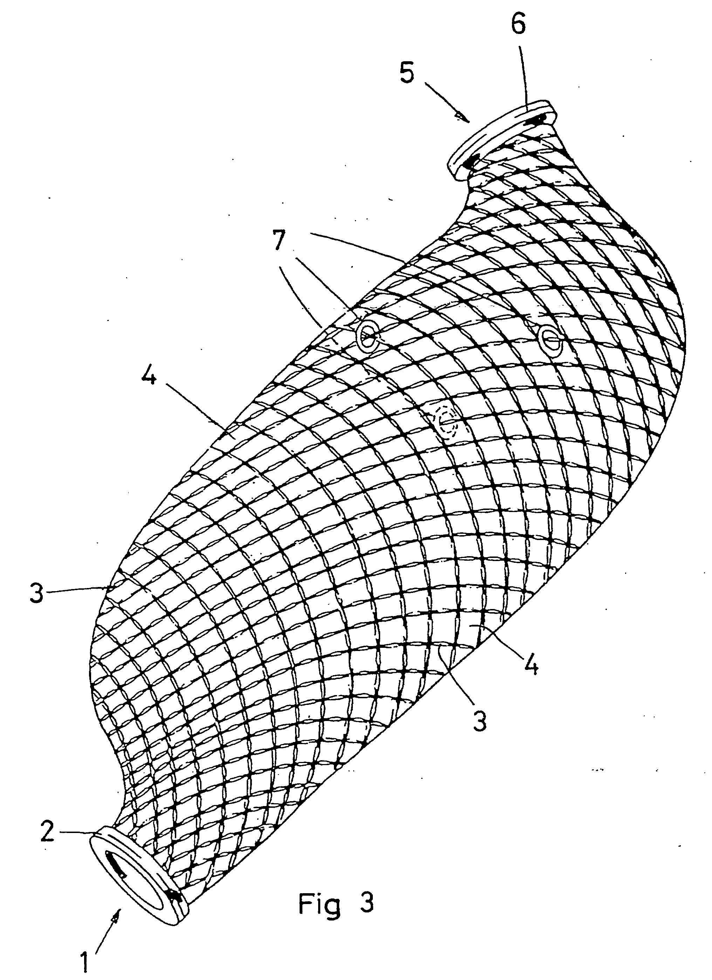

In Fig. 3, the receptacle is shown separately and approximately in the orientation it assumes after being operated into the abdominal cavity of the patient (Cf. Fig. 2). As was mentioned above, the receptacle comprises an inner container 4 or an ampoule which is produced from a lightly flexible, ideally somewhat elastic silicon material so that thereby the configuration of the inner receptacle may change in response to its level of filling. The outer casing 3 consists of a net of titanium threads which are so thin that they are relatively lightly flexible but naturally only stretchable to an extremely limited extent. This implies that the outer casing 3 lies fixed in an upper limit to which the ampoule 4 may be filled regardless of the inner pressure in it. The inner receptacle 4 and the outer casing 3 are free from one another and are movable in relation to one another apart from in the regions of the connection device and the emptying device. In Fig. 3, this situation is illustrated in that the receptacle has, in its upper/rear end, a rear connection 6 which serves for joining together the inner container or ampoule 4 and the outer casing 3. The rear connection 6 has a rear opening 5 via which the contents of the intestine pass into the receptacle. Correspondingly, the receptacle has, in its front/lower end, a front connection 2 with a front opening 1. The front connection 2 also serves for interconnection with or communication between the inner container or ampoule 4 and the outer casing 3. The lower or front connection 2 has a through-going opening via which the contents of the intestine pass from the receptacle via the emptying device and out to the ambient surroundings.

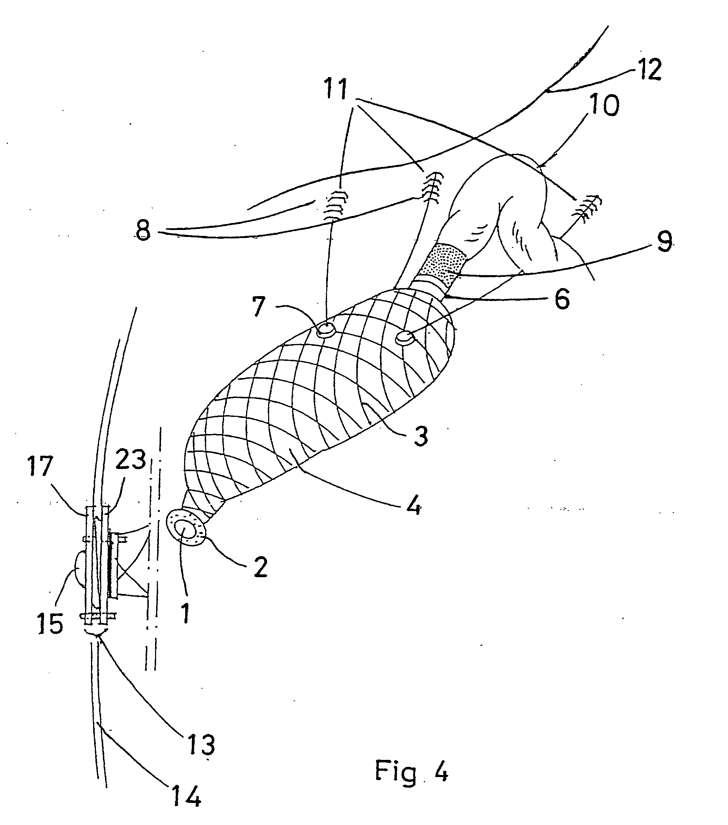

Fig. 4 illustrates how the receptacle, apart from via the emptying device, is interiorly secured in the abdominal cavity of the patient As will be apparent from both Fig. 3 and 4, the outer casing 3 has a number of coupling rings 7 which suitably enclose two mutually intersecting titanium threads in the outer casing. The coupling rings serve for securing anchorage threads or anchorage loops 8 which, as was mentioned above, have anchorages 11 interiorly in the abdominal cavity of the patient. Reference numeral 12 relates to diaphragms and reference numeral 13 to an abdominal wall system which realises an openable and closable passage through the abdominal wall 14 and which encloses the greater part of the emptying device.

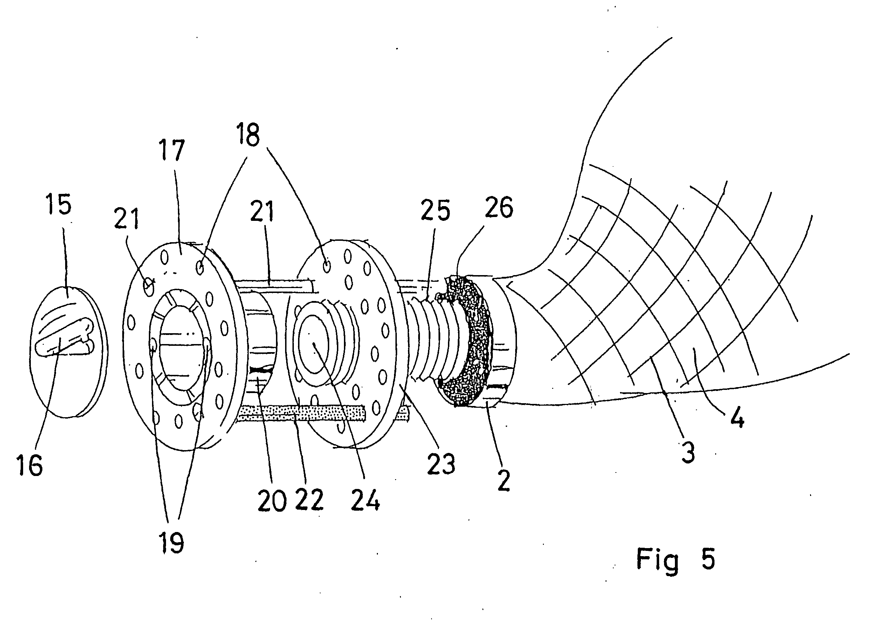

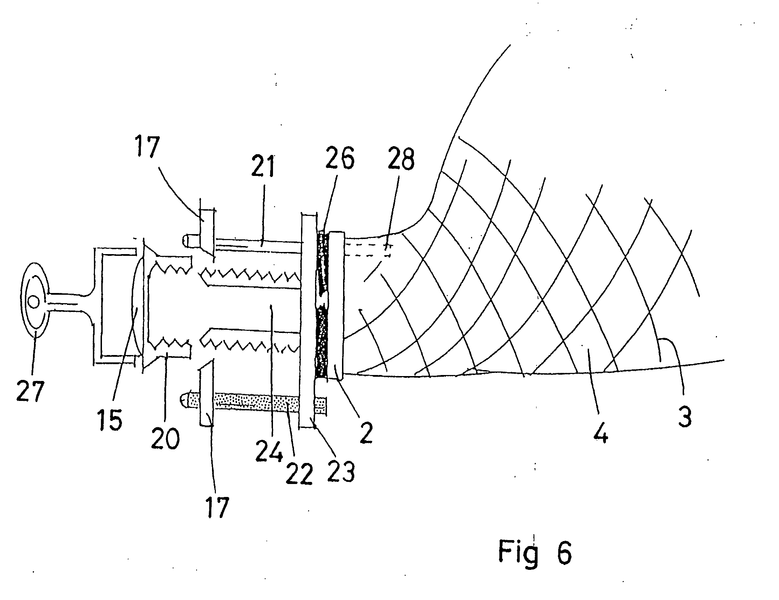

It will be apparent from Figs. 5 and 6 together that the abdominal wall system/the emptying device 13 includes a tubular, exteriorly threaded 25 part of a faecal emptying channel 24. The tubular part is connected via a threaded connection to the front connection 2 of the receptacle and extends through the inner fixing plate 23 and is also in threaded mesh therewith. On screwing of the inner fixing plate 23 in towards the connection 2, sealing will be realised by the intermediary of a sealing membrane 26 which is placed between the connection and the fixing plate.

The outer fixing plate 17 has a central, through-going aperture through which a fixing nut 20 may be passed into engagement with an outer end section of the emptying channel 24 and its exteriorly threaded section 25. The fixing nut 20 has a conical outer end member which cooperates with a corresponding conical hole in the outer fixing plate 17. For preventing mutual rotation between the fixing nut 20 and the outer fixing plate 17, this has two holes 19 via which rotation-impeding means may be inserted into cooperation with the fixing nut 20.

In its outer, outwardly facing end, the fixing nut 20 has engagement means for fixedly securing an emptying lid 15 which has a locking device 16.

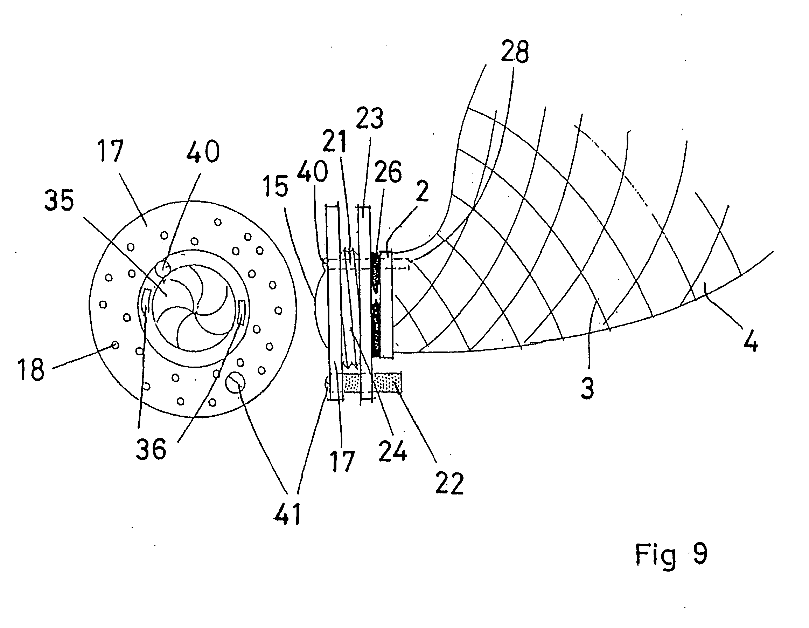

Through the two fixing plates 17 and 23, there extend, on the one hand, an evacuation tube 21 and, on the other hand, a communication tube 22. The evacuation tube discharges with its one, outer end on the outside of the outer fixing plate 17 and with its opposing, inner end, interiorly in the ampoule 4 at reference numeral 28. The communication tube 22 discharges with its outer end in an opening on the outside of the outer fixing plate 17 and with its opposing, inner end on the inside of the inner fixing plate 23, i.e. directly in the abdominal cavity of the patient. The discharge openings for the evacuation tube 21 and the communication tube 22 on the outside of the outer fixing plate 17 are shown in Fig. 9 at reference numerals 40 and 41, respectively. Further details will be presented below as regards the evacuation tube 21 and the communication tube 22.

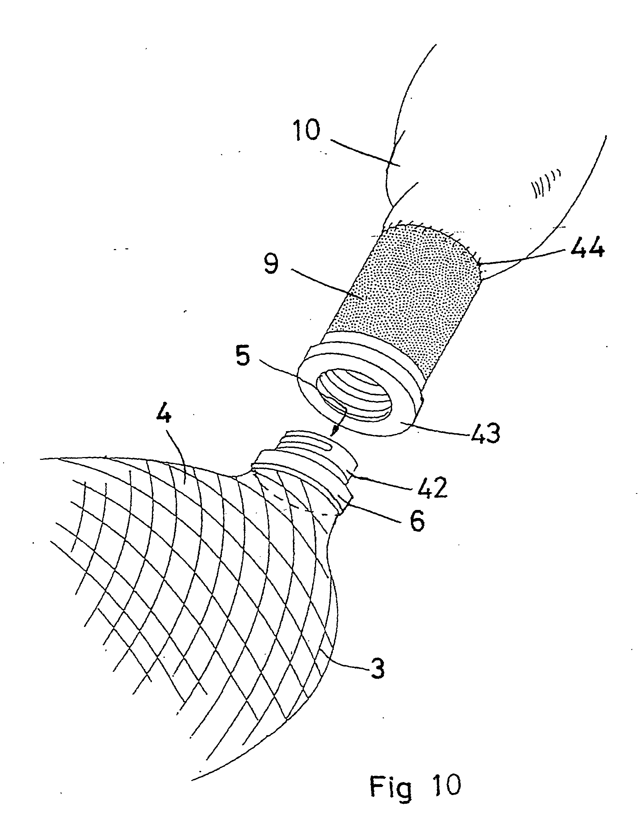

Fig. 10 shows the connection device with the rear connection 6 of the inner container or ampoule 4 and a coupling 42 fixedly secured therein and also having the through-going rear opening 5. The coupling 42 is intended for interconnection with a corresponding coupling or anchorage ring 43 which is located at the front/lower end of the interjacent member 9 which, at its upper, rear end, is secured via sutures 44 to the front, lower end of the intestine 10. The interjacent member 9 may suitably be produced from a fibrous or porous material such as Dacron® or Gore Tex®. The intestine 10 extends into the interjacent member 9 which may have a length of the order of magnitude of 5-8 cm. In order to facilitate the growth of intestinal epithelium in the interjacent member 9 and thereby improve the connection, the inside of the interjacent member is prepared with a thin matrix which is to constitute stroma for the growth of intestinal epithelium. This matrix consists of a stratum of collagen and/or fibrinonectrine. Growth factors may also be prepared in the matrix which stimulate the growth of intestinal epithelium. Thus, the intestinal epithelium will grow into the interjacent member 9 and form a safe and reliable connection with it.

The employment of the above-mentioned interjacent member 9 implies two advantages. First, it absorbs the intestinal peristalsis, i.e. the movement executed by the intestine for transporting the intestinal contents in a direction towards the receptacle. Secondly, the employment of the interjacent member 9 makes it possible that only one single operative intervention need be carried out since the intestine 10 is sutured direct to the interjacent member 9. This implies a direct connection which may be used immediately.

Fig. 9 shows the emptying device in the assembled and mounted state and it will be apparent that the evacuation tube 21 has a discharge opening 40 in the outer fixing plate 17, while the communication tube 22 has a corresponding opening 41. At the inner end of the evacuation tube, there is disposed a sensor which is located in the ampoule 4 and is in direct contact with its contents. The sensor is employed for registering changes of the pressure interiorly in the ampoule and emits a signal when the pressure reaches a certain critical level, which implies either that the discharge of gases from the ampoule must be put into effect, or that the ampoule must be emptied. In such a state, the sensor 28 emits a warning signal to the patient.

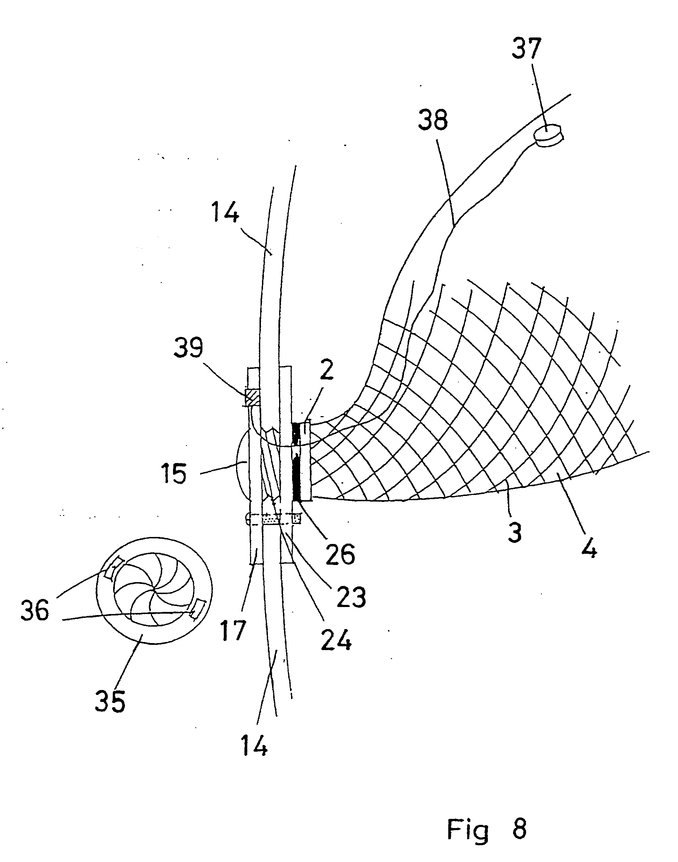

In the embodiment according to Fig. 8, a sensor 37 is placed higher up and further back in the ampoule 4 as well as interiorly in it. This sensor 37 is connected, via a conductor 38, to the outside of the outer fixing plate 17 where a battery ampoule 39 is placed for current supply, on the one hand, to the sensor 37 and, on the other hand, to the signal device which warns the patient when, for example, the receptacle ampoule is filled to approximately 90%.

Both Figs. 8 and 9 show, on the outside of the outer fixing plate 17, aperture anchorages 36 for the emptying catheter which is employed on emptying of the ampoule. In addition, Fig. 8 shows a valve 35 which is intended for closing the through-going emptying channel 24 when no emptying is to take place. The valve 35 may be constructed in accordance with the same principles as a so-called iris shutter or diaphragm and is operated by the emptying catheter via the anchorages 36.

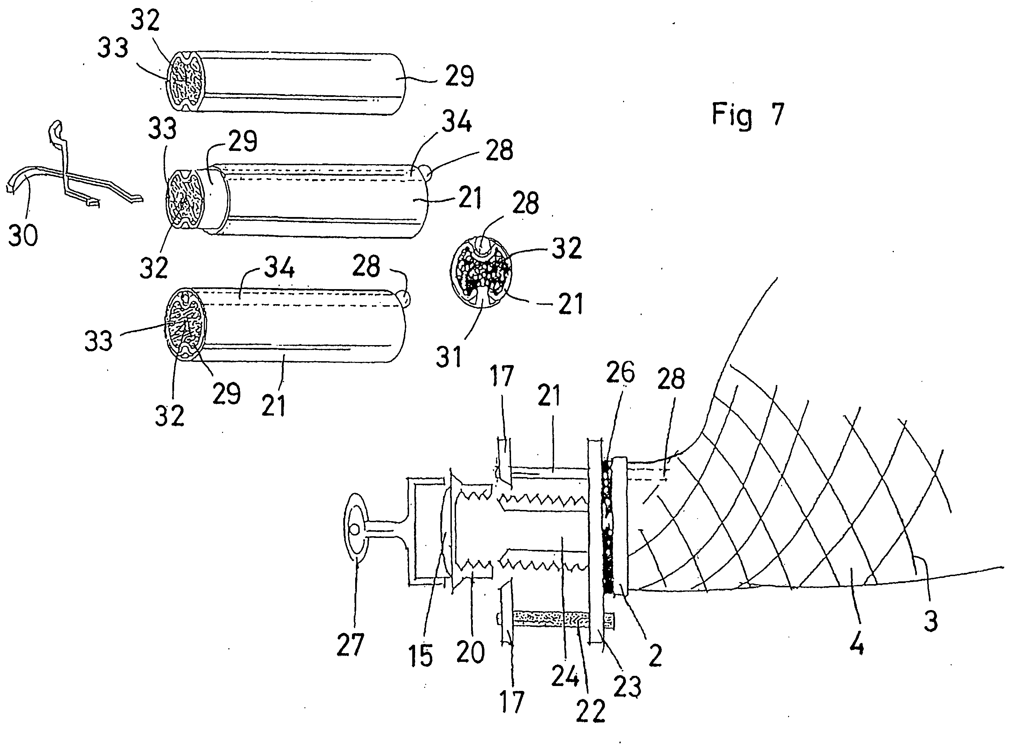

On release of gases from the interior of the ampoule 4 without simultaneous emptying of the intestinal contents therein, the evacuation tube 21 is employed. According to Fig. 7, the evacuation tube 21 is interiorly provided with a filter which is accessible on the outside of the outer fixing plate 17 beside the emptying valve 35. Replacement of filter may also be put into effect via the outer opening 40 (Fig. 9) of the tube 21. Via the same opening, replacement of the sensor which is placed at the inner end of the tube 21 may also be put into effect.

In an alternative embodiment, the filter is placed in an insert tube 29 which is insertible in and removable from the evacuation tube 21. The insert tube 29 has an inner heel 31 which prevents excessively long insertion of the insert tube 29. Interiorly, the insert tube is filled with a filter 32 and has recesses 33 for a gripping tool 30 by means of which the insert tube may be inserted into and removed from the evacuation tube 21.

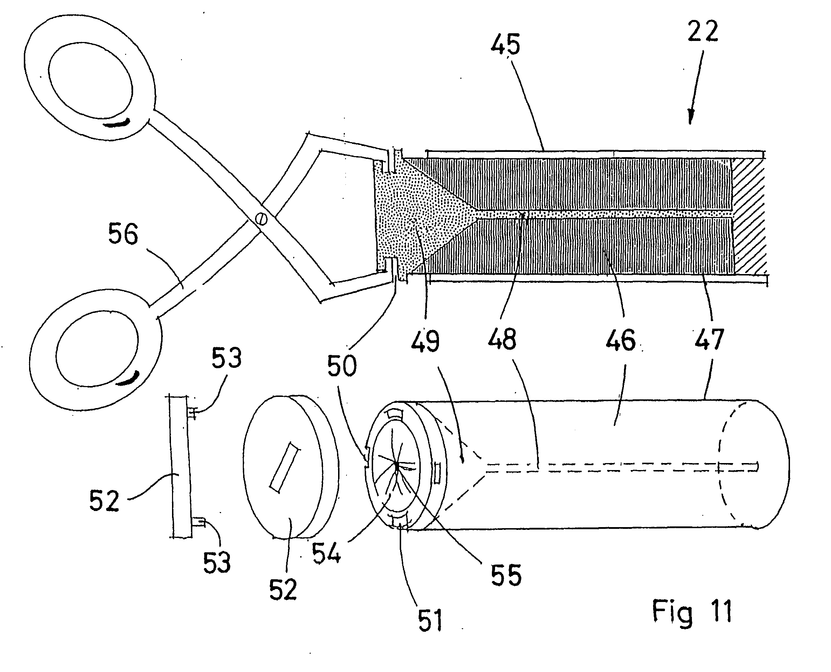

The communication tube 22 has, as was mentioned above (see Fig. 9), a discharge opening 41 in the outer fixing plate 17. The opposing end of the tube discharges interiorly in the abdominal cavity of the patient. It will be apparent from Fig. 11 that the communication tube 22 has an outer surface 45 and that this is provided with an insert tube 46 which has an outer surface 47 which connects to the inner surface in the communication tube. The insert tube 46 is insertible in and removable from the communication tube and, to this end, has gripping anchorages 50 in which forceps 56 may engage.

The insert tube 46 has a central, through-going injection passage 48 which, in the outer end, connects to a conical centring funnel 49 which makes it easier to centre, for example, an injection syringe for passage into the injection passage 48.

In the outer end, the insert tube 46 is covered by means of a membrane 54 which has a centring zone 55 in its centre. The outer end of the insert tube is further provided with anchorage recesses 51 for securing a lid 52 which, on its inner side, has gripping claws for fitting and locking in the above-mentioned recesses 51.

If larger instruments are to be passed through the communication tube 22 to the interior of the abdominal cavity, for example, for minor operative interventions, fibre optics or the like, the insert tube 46 is removed in its entirety.

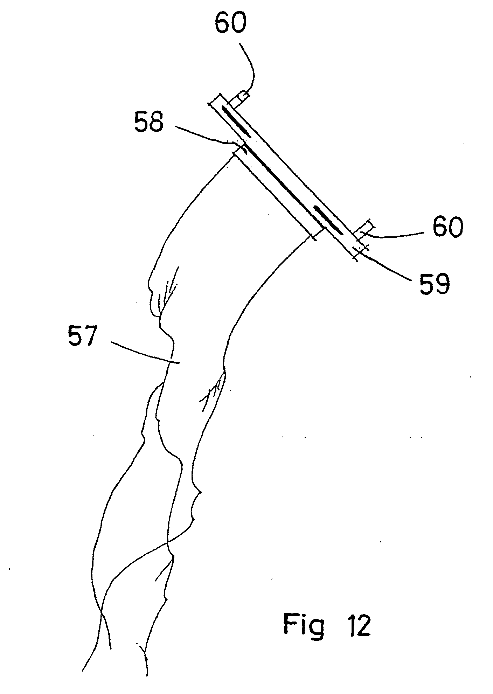

Fig. 12 shows an emptying catheter 57 which, in principle, consists of a readily flexible plastic hose of slight wall thickness. The upper end of the catheter is secured in an anchorage 58 which has a centring plate 59 with gripping claws 60 for cooperation with the opening anchorages 36 on the outer fixing plate 17. By cooperation of the gripping claws 60 and the above-mentioned opening anchorages 36, it is possible, on the one hand, to connect the catheter to the outer fixing plate and, on the other hand, to open and close the valve 35 which is disposed at the fixing plate.

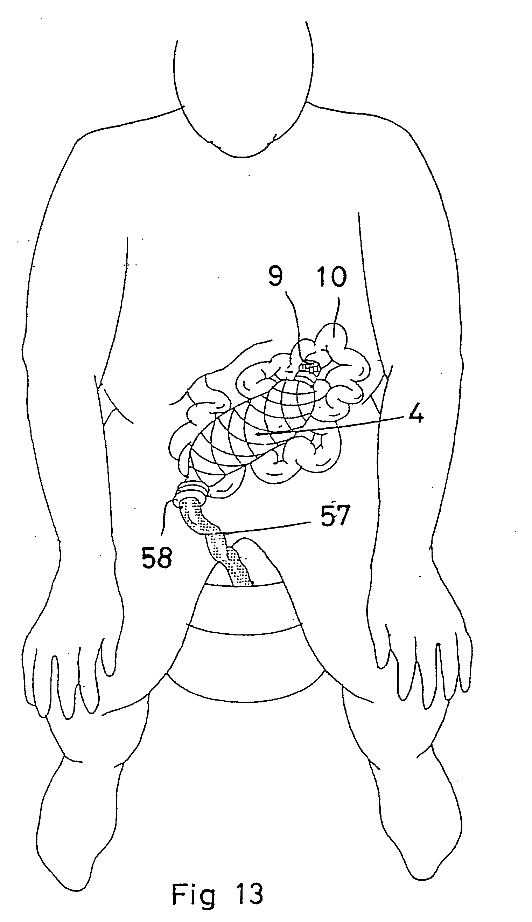

Fig. 13 shows how the emptying catheter has been secured at its anchorage 58 on the outer fixing plate 17 on the skin of the patient's abdomen. The Figure also illustrates how the catheter extends down into a toilet bowl.

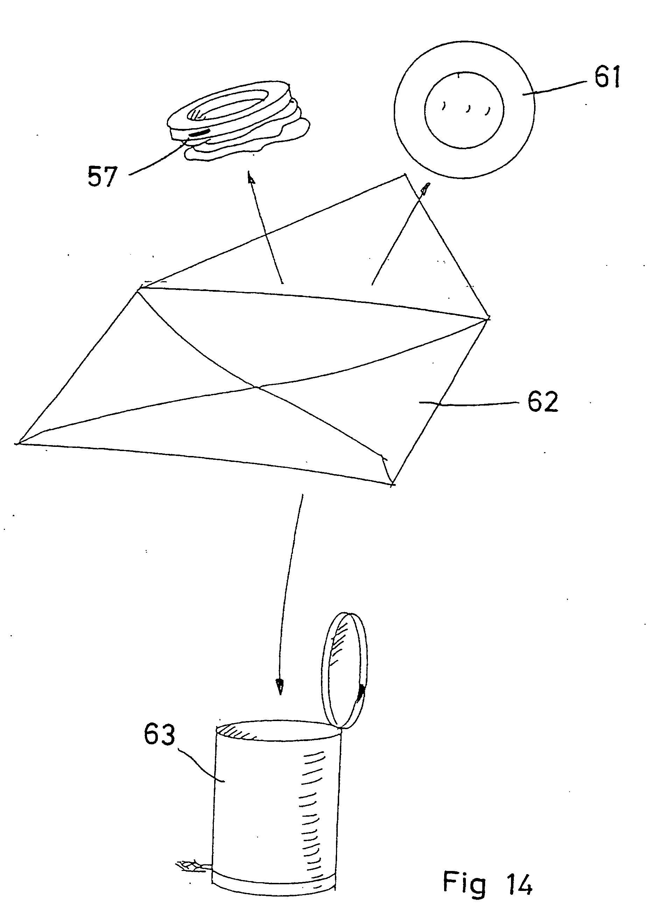

It will be apparent from Fig. 14 that the catheter 57 and a skin-coloured plaster 61 may suitably be packed in a single-use disposable package 62. On use, this package is opened and the catheter is removed and used, whereafter the plaster is placed over the fixing plate. Thereafter, the used catheter may once again be placed in the package 61 and the whole unit be deposited in a suitable refuse vessel 63.

In the foregoing, consideration has been given to the design of the two fixing plates 17 and 23. Suitably, these plates, but also the emptying channel 25, the evacuation tube 21 and the communication tube 22, are produced from titanium. In order to prevent necrosis in the abdominal wall, the two fixing plates are perforated and, as was mentioned above, have an adjustment capability towards and away from one another so that the abutment pressure against the abdominal wall 14 may thereby be modified.

On surgical implant of the emptying device in place, the outer fixing plate 17 is sutured in position on the skin of the abdominal wall. Further, the inner fixing plate 23 is sutured in place against the inner surface of the abdominal wall 14.

## Claims

1. A receptacle for the collection of intestinal contents and designed for surgical implant in the abdominal cavity of a patient who, post-operatively, lacks the whole or part of the small intestine, large intestine or the rectum, and comprising a connection device for connection to the intestine of the patient and an emptying device which realises a closable passage through the abdominal wall of the patient and is fixed therein, **characterised in that** the receptacle includes an inner container (4) or ampoule of a gas- and liquid-tight material, which is flexible and possibly somewhat elastic, and an outer casing (3) of a flexible but substantially unstretchable material.

2. The receptacle as claimed in Claim 1, **characterised in that** the ampoule (4) and the outer casing (3) are united to one another at the connection device and the emptying device.

3. The receptacle as claimed in Claim 1 or 2, **characterised in that** the outer casing (3) is of net configuration and consists of a metal.

4. The receptacle as claimed in Claim 3, **characterised in that** the metal is titanium.

5. The receptacle as claimed in any of Claims 1 to 4, **characterised in that** the ampoule (4) consists of a silicon material.

6. The receptacle as claimed in any of Claims 1 to 5, **characterised in that** the outer casing (3) has, in addition to the emptying device secured at the patient's abdominal wall (14), anchorage means (18, 11) for positionally fixing of the receptacle in the abdominal cavity of the patient.

7. The receptacle as claimed in any of Claims 1 to 6, **characterised in that** the connection device has, on its side of the connection (6) of the ampoule and the outer casing (3) facing away from the ampoule (4), a connection member or an interjacent member (9) designed for accommodating an end region of the intestine (10) and for securing thereof by means of sutures.

8. The receptacle as claimed in Claim 7, **characterised in that** the interjacent member (9) has, on its inside, a material for facilitating the growth of the intestine (10) in place in the interjacent member.

9. The receptacle as claimed in any of Claims 1 to 8, **characterised in that** the ampoule (4) and the outer casing (3) are free from one another apart from their connections (2, 6) at the emptying device and the connection device, respectively.

10. The receptacle as claimed in any of Claims 1 to 9, **characterised in that** the emptying device includes an outer fixing plate (17) for abutment against the skin of the patient's abdominal wall (14) and an inner fixing plate (23) for abutment against the inside of the patient's abdominal wall, a tubular passage section (24, 25) extending through the fixing plates and communicating, at its inner end, with the interior of the ampoule (4) and which, at its outer end, has an openable and closable valve (15).

11. The receptacle as claimed in Claim 10, **characterised in that** the mutual spacing of the fixing plates (17, 23) is adjustable by the intermediary of a screw arrangement (25).

12. The receptacle as claimed in Claim 10 or 11, **characterised in that** the fixing plates (17, 23) are perforated.

13. The receptacle as claimed in any of Claims 10 to 12, **characterised in that** there is disposed, through the fixing plates (17, 23), an evacuation tube (21) for gases, said evacuation tube communicating, with its inner end, with the interior of the ampoule (4) and which, at its outer end (40), has an openable closure device.

14. The receptacle as claimed in Claim 12, **characterised in that** a gas-permeable, replaceable filter (32) is disposed in the evacuation tube (21).

15. The receptacle as claimed in any of Claims 10 to 14, **characterised in that** there is disposed, through the fixing plates (17, 23), a communication tube (22) which, with its inner end, is in communication with the abdominal cavity and which, with its outer end (41), has an openable closure device.

## Patentansprüche

1. Behälter zum Sammeln von Darminhalt, der zur chirurgischen Implantation in die Bauchhöhle eines Patienten gestaltet ist, dem postoperativ der Dünndarm, der Dickdarm oder das Rektum ganz oder teilweise fehlen, enthaltend eine Anschlusseinrichtung zur Verbindung mit dem Darm des Patienten und eine Entleerungseinrichtung, die einen verschließbaren Durchgang durch die Bauchwand des Patienten schafft und darin fixiert ist, **dadurch gekennzeichnet, dass** der Behälter einen inneren Behälter (4) oder Ampulla aus einem gas- und flüssigkeitsdichten Material, der flexibel und möglicherweise in gewissem Ausmaß elastisch ist, und ein äußeres Gehäuse (3) aus einem flexiblen, jedoch im wesentlichen nicht dehnbaren Material enthält.

2. Behälter nach Anspruch 1,
**dadurch gekennzeichnet, dass** die Ampulla (4) und das äußere Gehäuse (3) an der Anschlusseinrichtung und der Entleerungseinrichtung miteinander vereint sind.

3. Behälter nach Anspruch 1 oder 2,
**dadurch gekennzeichnet, dass** das äußere Gehäuse (3) eine Netzkonfiguration hat und aus Metall besteht.

4. Behälter nach Anspruch 3,
**dadurch gekennzeichnet, dass** das Metall Titan ist.

5. Behälter nach einem der Ansprüche 1 bis 4,
**dadurch gekennzeichnet, dass** die Ampulla (4) aus einem Siliconmaterial besteht.

6. Behälter nach einem der Ansprüche 1 bis 5,
**dadurch gekennzeichnet, dass** das äußere Gehäuse (3) zusätzlich zu der an der Bauchwand (14) befestigten Entleerungseinrichtung Verankerungseinrichtungen (18, 11) zur Fixierung der Position des Behälters in der Bauchhöhle des Patienten aufweist.

7. Behälter nach einem der Ansprüche 1 bis 6,
**dadurch gekennzeichnet, dass** die Anschlusseinrichtung auf ihrer Seite der Verbindung (6) der Ampulla und des äußeren Gehäuses (3), die von der Ampulla (4) wegweist, ein Verbindungselement oder ein Zwischenelement (9) aufweist, das zur Unterbringung eines Endbereichs des Darmes (10) und zur Befestigung desselben mittels Nähten gestaltet ist.

8. Behälter nach Anspruch 7,
**dadurch gekennzeichnet, dass** das Zwischenelement (9) an seiner Innenseite ein Material zum Erleichtern des Wachstums des Darmes (10) an der Position in dem Zwischenelement hat.

9. Behälter nach einem der Ansprüche 1 bis 8,
**dadurch gekennzeichnet, dass** die Ampulla (4) und das äußere Gehäuse (3) abgesehen von ihren Verbindungen (2, 6) an der Entleerungseinrichtung bzw. an der Anschlusseinrichtung frei voneinander sind.

10. Behälter nach einem der Ansprüche 1 bis 9,
**dadurch gekennzeichnet, dass** die Entleerungseinrichtung eine äußere Befestigungsplatte (17), die an der Haut der Bauchwand (14) des Patienten anliegt, und eine innere Befestigungsplatte (23), die an der Innenseite der Bauchwand des Patienten anliegt, sowie einen rohrförmigen Durchgangsabschnitt (24, 25) aufweist, der durch die Befestigungsplatten verläuft und an seinem inneren Ende mit dem Inneren der Ampulla (4) in Verbindung steht und der an seinem äußeren Ende ein zu öffnendes und zu schließendes Ventil (15) aufweist.

11. Behälter nach Anspruch 10,
**dadurch gekennzeichnet, dass** die gegenseitige Beabstandung der Befestigungsplatten (17, 23) mittels einer Gewindeanordnung (25) einstellbar ist.

12. Behälter nach Anspruch 10 oder 11,
**dadurch gekennzeichnet, dass** die Befestigungsplatten (17, 23) perforiert sind.

13. Behälter nach einem der Ansprüche 10 bis 12,
**dadurch gekennzeichnet, dass** durch die Befestigungsplatten (17, 23) ein Entleerungsschlauch (21) für Gase angeordnet ist, welcher Entleerungsschlauch mit seinem inneren Ende mit dem Inneren der Ampulla (4) in Verbindung steht und an seinem äußeren Ende (40) eine Verschlusseinrichtung hat, die geöffnet werden kann.

14. Behälter nach Anspruch 12,
**dadurch gekennzeichnet, dass** ein gaspermeabler, austauschbarer Filter (32) in dem Entleerungsschlauch (21) angeordnet ist.

15. Behälter nach einem der Ansprüche 10 bis 14,
**dadurch gekennzeichnet, dass** durch die Befestigungsplatten (17, 23) ein Verbindungsschlauch (22) angeordnet ist, der mit seinem inneren Ende mit der Bauchhöhle in Verbindung steht und der an seinem äußeren Ende (41) eine Verschlusseinrichtung hat, die geöffnet werden kann.

## Revendications

1. Réceptacle pour recueillir un contenu intestinal et conçu pour implantation chirurgicale dans la cavité abdominale d'un patient à qui il manque, de façon post-opératoire, la totalité ou une partie de l'intestin grêle, du gros intestin ou du rectum, et comprenant un dispositif de connexion pour raccordement à l'intestin du patient et un dispositif de vidange qui constitue un passage pouvant se fermer à travers la paroi abdominale du patient et est fixé dans celle-ci, **caractérisé en ce que** le réceptacle comprend un récipient intérieur (4) ou une ampoule en une matière étanche aux gaz et aux liquides, qui est souple et éventuellement un peu élastique, et une enveloppe extérieure (3) en une matière souple mais sensiblement non extensible.

2. Réceptacle selon la revendication 1, **caractérisé en ce que** l'ampoule (4) et l'enveloppe extérieure (3) sont reliées l'une à l'autre à l'endroit du dispositif de connexion et du dispositif de vidange.

3. Réceptacle selon la revendication 1 ou 2, **caractérisé en ce que** l'enveloppe extérieure (3) a une configuration en filet et est en métal.

4. Réceptacle selon la revendication 1, **caractérisé en ce que** le métal est le titane.

5. Réceptacle selon une quelconque des revendications 1 à 4, **caractérisé en ce que** l'ampoule (4) est en une matière de type silicone.

6. Réceptacle selon une quelconque des revendications 1 à 5, **caractérisé en ce que** l'enveloppe extérieure (3) comporte, en plus du dispositif de vidange fixé à la paroi abdominale (14) du patient, des moyens d'ancrage (18, 11) pour fixer en position le réceptacle dans la cavité abdominale du patient.

7. Réceptacle selon une quelconque des revendications 1 à 6, **caractérisé en ce que** le dispositif de connexion comporte, sur son côté de la connexion (6) de l'ampoule et de l'enveloppe extérieure (3) qui est tourné à l'opposé de l'ampoule (4), un élément de connexion ou un élément d'interposition (9) prévu pour recevoir une région d'extrémité de l'intestin (10) et pour fixer celle-ci au moyen de sutures.

8. Réceptacle selon la revendication 7, **caractérisé en ce que** l'élément d'interposition (9) comporte, sur son côté intérieur, une matière qui facilite la croissance de l'intestin (10) en place dans l'élément d'interposition.

9. Réceptacle selon une quelconque des revendications 1 à 8, **caractérisé en ce que** l'ampoule (4) et l'enveloppe extérieure (3) sont mutuellement indépendantes en dehors de leurs connexions (2, 6) à l'endroit du dispositif de vidange et du dispositif de connexion, respectivement.

10. Réceptacle selon une quelconque des revendications 1 à 9, **caractérisé en ce que** le dispositif de vidange comprend une plaque de fixation extérieure (17) venant en butée contre la peau de la paroi abdominale du patient (14) et une plaque de fixation intérieure (23) venant en butée contre l'intérieur de la paroi abdominale du patient, un élément de passage tubulaire (24, 26) traversant les plaques de fixation et communiquant, à son extrémité intérieure, avec l'intérieur de l'ampoule (4) et comportant, à son extrémité extérieure, une valve (15) pouvant s'ouvrir et se fermer.

11. Réceptacle selon la revendication 10, **caractérisé en ce que** l'espacement mutuel des plaques de fixation (17, 23) est réglable par l'intermédiaire d'un dispositif à vis (25).

12. Réceptacle selon la revendication 10 ou 11, **caractérisé en ce que** les plaques de fixation (17, 23) sont perforées.

13. Réceptacle selon une quelconque des revendications 10 à 12, **caractérisé en ce qu'**il est prévu, à travers les plaques de fixation (17, 23), un tube d'évacuation (21) pour les gaz, le dit tube d'évacuation communiquant, par son extrémité intérieure, avec l'intérieur de l'ampoule (4) et comportant, à son extrémité extérieure (40), un dispositif de fermeture pouvant s'ouvrir.

14. Réceptacle selon la revendication 12, **caractérisé en ce qu'**un filtre remplaçable perméable aux gaz (32) est disposé dans le tube d'évacuation (21).

15. Réceptacle selon une quelconque des revendications 10 à 14, **caractérisé en ce qu'**il est prévu, à travers les plaques de fixation (17, 23), un tube de communication (22) qui communique, par son extrémité intérieure, avec la cavité abdominale et qui comporte, à son extrémité extérieure (41), un dispositif de fermeture pouvant s'ouvrir.
